(19) Europäisches Patentamt / European Patent Office / Office européen des brevets

(11) **EP 2 628 449 B1**

(12) **EUROPEAN PATENT SPECIFICATION**

(45) Date of publication and mention
of the grant of the patent:
**20.05.2020 Bulletin 2020/21**

(51) Int Cl.:
***A61B 8/00*** (2006.01)

(21) Application number: **13155425.5**

(22) Date of filing: **15.02.2013**

(54) **Ultrasound apparatus and method of generating ultrasound image**

Ultraschallvorrichtung und Verfahren zur Erzeugung eines Ultraschallbildes

Appareil à ultrasons et procédé de génération d'image ultrasonore

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.02.2012 US 201261600062 P**
**10.07.2012 KR 20120075171**

(43) Date of publication of application:
**21.08.2013 Bulletin 2013/34**

(73) Proprietor: **Samsung Electronics Co., Ltd.**
**Gyeonggi-do 443-742 (KR)**

(72) Inventors:
- **Shim, Hwan**
  **Yongin-si (KR)**
- **Lim, Hyung-joon**
  **Seoul (KR)**
- **Cheon, Byeong-geun**
  **Anyang-si (KR)**
- **Kim, Young-tae**
  **Seongnam-si (KR)**

(74) Representative: **Appleyard Lees IP LLP**
**15 Clare Road**
**Halifax HX1 2HY (GB)**

(56) References cited:
US-A- 4 800 891       US-A1- 2003 100 832
US-A1- 2003 212 329   US-B1- 6 464 637

- **MANIATIS T A ET AL: "Two-dimensional velocity reconstruction strategies for color flow doppler ultrasound images", ULTRASOUND IN MEDICINE AND BIOLOGY, NEW YORK, NY, US, vol. 20, no. 2, 1 January 1994 (1994-01-01), pages 137-145, XP026409531, ISSN: 0301-5629 [retrieved on 1994-01-01]**
- **DUNMIRE B ET AL: "Cross-beam vector Doppler ultrasound for angle-independent velocity measurements", ULTRASOUND IN MEDICINE AND BIOLOGY, NEW YORK, NY, US, vol. 26, no. 8, 1 October 2000 (2000-10-01), pages 1213-1235, XP004295674, ISSN: 0301-5629, DOI: 10.1016/S0301-5629(00)00287-8**
- **BUROV V A ET AL: "Reconstruction of the distribution pattern for the bloodstream velocity vector in the acoustic tomography procedure", ACOUSTICAL PHYSICS, NAUKA/INTERPERIODICA, MO, vol. 52, no. 5, 1 September 2006 (2006-09-01), pages 518-532, XP019411966, ISSN: 1562-6865, DOI: 10.1134/S1063771006050046**
- **OVERBECK J R ET AL: "Vector doppler: Accurate measurement of blood velocity in two dimensions", ULTRASOUND IN MEDICINE AND BIOLOGY, NEW YORK, NY, US, vol. 18, no. 1, 1 January 1992 (1992-01-01), pages 19-31, XP026450894, ISSN: 0301-5629 [retrieved on 1992-01-01]**

**Description**

BACKGROUND

1. Field

The present disclosure relates to a method and apparatus for generating an ultrasound image and, more particularly, to a method and apparatus for generating a Doppler image of an object.

2. Description of the Related Art

Ultrasound apparatuses are used to monitor the internal structure of an organic body. Ultrasound apparatuses are non-invasive test devices and show structural details, tissues, and fluidic flows in the body.

An ultrasound apparatus may display the flow of blood or movement of tissues as a color Doppler image or a spectral Doppler image, and a user may view the color Doppler image or the spectral Doppler image to check the motion of heart valves, a blood velocity or a blood flow rate in blood vessels, or the like.

Since the color Doppler image and the spectral Doppler image include information about a velocity magnitude and a velocity direction of a blood flow or tissues, a method of accurately measuring the information is required.

Maniatis et al, "Two-dimensional velocity reconstruction strategies for color flow Doppler ultrasound images" (1994), as cited in the search report, discloses a strategy for calculating a 2-D velocity vector flow field from colour flow Doppler ultrasound images obtained from two or three steering angles.

Dunmire et al: "Cross-beam vector Doppler ultrasound for angle-independent velocity measurements" (2000), as cited in the search report, presents a review of cross-beam vector Doppler ultrasound systems, including a system which combines received signals from three steering angles.

US Patent Application No. 2003/100832 A1 discloses a 3D Doppler ultrasonic imaging system, in which images are calculated by combining 2D measurements received from pairs of apertures aligned along common axes. Burov et al: "Reconstruction of the distribution pattern for the bloodstream velocity vector in the acoustic tomography procedure" (2006), as cited in the search report, details an ultrasonic tomography method for imaging a whole organ.

US Patent Application No. 2003/212329 A1 discloses an angle-independent Doppler system, in which the receiving elements are arranged radially around the transmitter.

US Patent No. 4800891A discloses a method for estimating the velocity of a moving target using Doppler signals.

SUMMARY

The invention is defined in the independent claims 1,8 and 15.

The present disclosure provides an ultrasound apparatus and a method of generating an ultrasound image which are capable of accurately measuring vector information of an object.

The present disclosure also provides an ultrasound apparatus and a method of generating an ultrasound image which are capable of providing a reliable color Doppler image or a reliable spectral Doppler image to a user.

According to the present invention there is provided an apparatus and method as set forth in the appended claims. Other features of the invention will be apparent from the dependent claims, and the description which follows.

According to an aspect of an exemplary embodiment, there is provided a method of generating an ultrasound image, the method including transmitting an ultrasound signal to a predetermined point included in an object and receiving at least three response signals reflected from the predetermined point; extracting a plurality of response signal pairs by combining the received at least three response signals two-by-two; obtaining a plurality of pieces of prediction vector information, each of the pieces of prediction vector information indicating a prediction velocity magnitude and a prediction velocity direction of the predetermined point, based on reception angles and Doppler frequencies of the response signals included in the plurality of response signal pairs; and determining vector information indicating a velocity magnitude and a velocity direction of the predetermined point, based on the plurality of pieces of prediction vector information.

The determining of the vector information may include determining the vector information based on a linear vector sum of the plurality of pieces of prediction vector information.

The determining of the vector information may include determining any one of the plurality of prediction velocity directions included in the plurality of pieces of prediction vector information, as the velocity direction of the predetermined point; and determining a linear sum of the plurality of prediction velocity magnitudes included in the plurality of pieces of prediction vector information, as the velocity magnitude of the predetermined point.

The method may further include determining vector information of a plurality of additional points located within a preset distance from the predetermined point.

The determining of the vector information may include compensating for the velocity direction of the predetermined point by applying a smoothing filter to velocity directions of the plurality of additional points; and determining the compensated velocity direction as the velocity direction of the predetermined point.

The determining of the vector information may include compensating for the velocity magnitude of the predetermined point by applying a smoothing filter to velocity magnitudes of the plurality of additional points; and determining the compensated velocity magnitude as the velocity magnitude of the predetermined point.

The transmitting of the ultrasound signal may include transmitting the ultrasound signal a plurality of times and receiving each of the at least three response signals a plurality of times, a plurality of the same received response signal forming ensemble signals for the respective response signal, and the obtaining of the plurality of pieces of prediction vector information may include obtaining a Doppler frequency of each response signal included in the plurality of response signal pairs by using autocorrelation between the ensemble signals of the response signals.

According to another aspect of an exemplary embodiment, there is provided a method of generating an ultrasound image, the method including transmitting an ultrasound signal to a point included in an object and receiving a response signal reflected from the point; determining vector information indicating a velocity magnitude of the point, based on a reception angle and a Doppler frequency of the response signal; and displaying a color Doppler image of the object by mapping the velocity magnitude of the point to a color scale.

The method may further include determining vector information including a velocity direction of a plurality of points included in the object; estimating an overall velocity direction of the object by using the velocity directions of the plurality of points; and displaying the color Doppler image of the object by mapping angle differences between the velocity directions of the plurality of points and the overall velocity direction of the object to a color scale.

The method may further include determining vector information including a velocity magnitude of a plurality of points included in the object, and the displaying of the color Doppler image of the object may include mapping a velocity magnitude of a point having a first velocity direction component, from among the plurality of points, to a first color and mapping a velocity magnitude of a point having a second velocity direction component opposite to the first velocity direction component, from among the plurality of points, to a second color.

According to another aspect of an exemplary embodiment, there is provided a method of generating an ultrasound image, the method including dividing an object into a plurality of regions; transmitting an ultrasound signal to the plurality of regions and receiving response signals reflected from the plurality of regions; measuring power levels or Doppler frequencies of the received response signals; and determining a monitoring region from among the plurality of regions by using the power levels or the Doppler frequencies of the received response signals.

The determining of the monitoring region may include arbitrarily setting an initial region from among the plurality of regions; and determining one of the regions reflecting response signals having Doppler frequencies in a first predetermined range, which is closest to the initial region, as the monitoring region.

The determining of the monitoring region may include determining one of the regions reflecting a response signal having a Doppler frequency in a second predetermined range, as the monitoring region.

The determining of the monitoring region may include arbitrarily setting an initial region from among the plurality of regions; and determining one of the regions reflecting response signals having power levels in a third predetermined range, which is closest to the initial region, as the monitoring region.

The determining of the monitoring region may include determining one of the regions reflecting a response signal having a power level in a fourth predetermined range, as the monitoring region.

The determining of the monitoring region may include determining one of the regions reflecting a response signal having a power level in a fifth predetermined range from among the response signals that pass through a clutter filter, as the monitoring region.

The method may further include adjusting a region of interest in such a way that the monitoring region is included in the region of interest.

The method may further include adjusting a range gate in such a way that the range gate is located in the monitoring region.

The method may further include measuring power levels or Doppler frequencies of response signals reflected from a plurality of the regions located within a preset distance from the monitoring region; obtaining a modeled line by applying a fitting method to the power levels or the Doppler frequencies of the response signals reflected from the plurality of regions; determining a blood vessel direction of the object based on the obtained modeled line; and adjusting a steering angle of the ultrasound signal in such a way that an angle between the blood vessel direction and an incident direction of the ultrasound signal transmitted to the plurality of regions is a predetermined angle.

According to another aspect of an exemplary embodiment, there is provided an ultrasound apparatus including a probe which transmits an ultrasound signal to a predetermined point included in an object and receives at least three response signals reflected from the predetermined point; an extraction unit which extracts a plurality of response signal pairs by combining the received at least three response signals two-by-two; a vector information obtaining unit which obtains a plurality of pieces of prediction vector information, each of the pieces of prediction vector information indicating a prediction velocity magnitude and a prediction velocity direction of the predetermined point, based on reception angles and Doppler frequencies of response signals included in the plurality of response signal pairs; and a vector information determination unit which determines vector information indicating a velocity magnitude and a velocity direction of the predetermined point, based on the plurality of pieces of prediction vector information.

The vector information determination unit may determine the vector information by using a linear vector sum of the plurality of pieces of prediction vector information.

The vector information determination unit may determine any one of the plurality of prediction velocity directions included in the plurality of pieces of prediction vector information, as the velocity direction of the predetermined point, and may determine a linear sum of the plurality of prediction velocity magnitudes included in the plurality of pieces of prediction vector information, as the velocity magnitude of the predetermined point.

The vector information determination unit may determine vector information of a plurality of additional points located within a preset distance from the predetermined point.

The vector information determination unit may compensate for the velocity direction of the predetermined point by applying a smoothing filter to velocity directions of the plurality of additional points, and may determine the compensated velocity direction as the velocity direction of the predetermined point.

The vector information determination unit may compensate for the velocity magnitude of the predetermined point by applying a smoothing filter to velocity magnitudes of the plurality of additional points, and may determine the compensated velocity magnitude as the velocity magnitude of the predetermined point.

The probe may transmit the ultrasound signal a plurality of times and receive each of the at least three response signals a plurality of times, a plurality of the same received response signal forming ensemble signals for the respective response signal, and the vector information obtaining unit may obtain a Doppler frequency of each response signal included in the plurality of response signal pairs by using autocorrelation between the ensemble signals of the response signals.

According to another aspect of an exemplary embodiment, there is provided an ultrasound apparatus including a probe which transmits an ultrasound signal to a point included in an object and receives a response signal reflected from the point; a vector information determination unit which determines vector information indicating a velocity magnitude of the point, based on a reception angle and a Doppler frequency of the response signal; and a display unit which displays a color Doppler image of the object by mapping the velocity magnitude of the point to a color scale.

The vector information determination unit may determine vector information including velocity directions of a plurality of points included in the object and estimates an overall velocity direction of the object by using the velocity directions of the plurality of points, and the display unit may display the color Doppler image of the object by mapping angle differences between the velocity directions of the plurality of points and the overall velocity direction of the object to a color scale.

The vector information determination unit may determine vector information including a vector magnitude of a plurality of points included in the object, and the display unit may map a velocity magnitude of a point having a first velocity direction component, from among the plurality of points, to a first color and may map a velocity magnitude of a point having a second velocity direction component opposite to the first velocity direction component, from among the plurality of points, to a second color.

According to another aspect of an exemplary embodiment, there is provided an ultrasound apparatus including a region dividing unit which divides an object into a plurality of regions; a probe which transmits an ultrasound signal to the plurality of regions and receives response signals reflected from the plurality of regions; a measurement unit which measures power levels or Doppler frequencies of the received response signals; and a control unit which determines a monitoring region from among the plurality of regions by using the power levels or the Doppler frequencies of the received response signals.

The control unit arbitrarily may set an initial region from among the plurality of regions, and may determine one of the regions reflecting response signals having Doppler frequencies in a first predetermined range, which is closest to the initial region, as the monitoring region.

The control unit may determine one of the regions reflecting a response signal having a Doppler frequency in a second predetermined range, as the monitoring region.

The control unit may arbitrarily set an initial region from among the plurality of regions, and may determine one of the regions reflecting response signals having power levels in a third predetermined range, which is closest to the initial region, as the monitoring region.

The control unit may determine one of the regions reflecting a response signal having a power level in a fourth predetermined range, as the monitoring region.

The control unit may determine one of the regions reflecting a response signal having a power level in a fifth predetermined range from among the response signals that pass through a clutter filter, as the monitoring region.

The control unit may adjust a region of interest in such a way that the monitoring region is included in the region of interest.

The control unit may adjust a range gate in such a way that the range gate is located in the monitoring region.

The measurement unit may measure power levels or Doppler frequencies of response signals reflected from a plurality of the regions located within a preset distance from the monitoring region, and the control unit may determine a blood vessel direction of the object based on a modeled line obtained by applying a fitting method to the power levels or the Doppler frequencies of the response signals reflected from the plurality of regions, and may adjust a steering angle of the ultrasound signal in such a way that an angle between the blood vessel direction and an incident direction of the ultrasound signal transmitted to the plurality of regions is a predetermined angle.

According to another aspect of an exemplary embodiment, there is provided a non-transitory computer-readable recording medium having recorded thereon a computer program for executing the method according to exemplary embodiments.

According to another aspect of an exemplary embodiment, there is provided an ultrasound apparatus including a probe which transmits an ultrasound signal to a point of an object and receives a plurality of response signals reflected back from the point, and a vector information determiner which determines a velocity direction and a velocity magnitude of the point based on the response signals, wherein, for each of the plurality of response signals, an angle between a reflection direction of the response signal and a velocity direction of the point is less than or greater than 90°.

## BRIEF DESCRIPTION OF THE DRAWINGS

The above and other features and advantages of the present disclosure will become more apparent by describing in detail exemplary embodiments thereof with reference to the attached drawings in which:

FIG. 1 is a schematic diagram for describing a general method of obtaining a velocity magnitude of an object;
FIG. 2 is a block diagram of an ultrasound apparatus according to an exemplary embodiment;
FIG. 3 is a schematic diagram showing an ultrasound signal being transmitted to a predetermined point of an object and response signals being received from the predetermined point using the ultrasound apparatus illustrated in FIG. 2;
FIG. 4 is a schematic diagram for describing a method of obtaining vector information of the predetermined point of the object using the ultrasound apparatus illustrated in FIG. 2;
FIG. 5A is a schematic diagram showing vector information of a plurality of points of the object obtained using the ultrasound apparatus illustrated in FIG. 2;
FIG. 5B is a schematic diagram showing a result of applying a smoothing filter to the vector information illustrated in FIG. 5A;
FIG. 6 is a block diagram of an ultrasound apparatus according to another exemplary embodiment;
FIGS. 7A and 7B are color Doppler images in which vector information of a plurality of points of an object is mapped to a color scale;
FIG. 8 is a flowchart of a method of generating an ultrasound image, according to an exemplary embodiment;
FIG. 9 is a block diagram of an ultrasound apparatus according to another exemplary embodiment;
FIG. 10 is a schematic diagram for describing a method of determining a monitoring region from among a plurality of regions of an object, using the ultrasound apparatus illustrated in FIG. 9;
FIG. 11 is a schematic diagram for describing another method of determining a monitoring region from among a plurality of regions of an object, using the ultrasound apparatus illustrated in FIG. 9;
FIG. 12 is a schematic diagram for describing a method of determining a location of a blood vessel included in an object, using the ultrasound apparatus illustrated in FIG. 9;
FIG. 13 is a schematic diagram for describing a method of adjusting a steering angle of an ultrasound signal, using the ultrasound apparatus illustrated in FIG. 9; and
FIG. 14 is a flowchart of a method of generating an ultrasound image, according to another exemplary embodiment.

## DETAILED DESCRIPTION OF THE INVENTION

The present disclosure will now be described more fully with reference to the accompanying drawings, in which exemplary embodiments are shown. The exemplary embodiments may, however, be embodied in many different forms and should not be construed as being limited to the exemplary embodiments set forth herein; rather, these exemplary embodiments are provided so that this disclosure will be thorough and complete, and will fully convey the concept of the exemplary embodiments to one of ordinary skill in the art. Like reference numerals in the drawings denote like elements.

The term "unit" in the exemplary embodiments of the present disclosure may refer to a software component or a hardware component, such as a field-programmable gate array (FPGA) or an application-specific integrated circuit (ASIC), that performs a specific function. However, the term "unit" is not limited to software or hardware. The "unit" may be formed so as to be stored in an addressable storage medium, or may be formed so as to operate one or more processors. Thus, for example, the term "unit" may refer to components such as software components, object-oriented software components, class components, and task components, processes, functions, properties, procedures, subroutines, segments of program codes, drivers, firmware, micro codes, circuits, data, databases, data structures, tables, arrays, and parameters. Functions provided by elements and "units" may be combined in a smaller number of elements and "units" or may be separated into additional elements and "units".

According to exemplary embodiments, the term 'vector information' refers to a vector value indicating a velocity magnitude and a velocity direction, and the term 'velocity magnitude' refers to a scalar value not indicating a direction of a velocity but indicating only a magnitude of the velocity. According to exemplary embodiments, the term 'velocity direction' refers to a direction in which a certain point of an object moves at an arbitrary time.

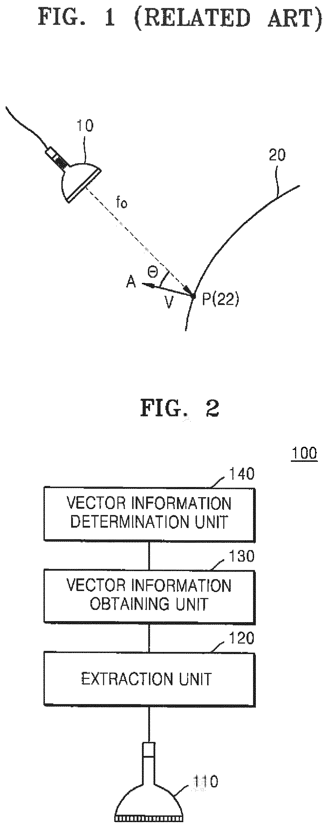

FIG. 1 is a schematic diagram for describing a general method of obtaining a velocity magnitude of an object 20.

In the present disclosure, an 'object' refers to a certain body part of which an ultrasound image is to be obtained, e.g., tissue, blood, or an organ.

A predetermined point P (22) of the object moves at a velocity magnitude v and in a velocity direction A. A probe 10 of an ultrasound apparatus transmits an ultrasound signal having a frequency f0 to the predetermined point P 22 and receives a response signal reflected from the predetermined point P (22). Based on the Doppler effect, a frequency of the response signal varies according to the velocity magnitude v of the predetermined point P (22) and an angle $\theta$ between an incident direction of the ultrasound signal and the velocity direction A of the predetermined point P (22), and a difference between the frequency of the ultrasound signal transmitted to the predetermined point P (22) and the frequency of the response signal is referred to as a Doppler frequency. Also, a signal having the Doppler frequency is referred to as a Doppler signal. The velocity magnitude v of the predetermined point P (22) may be obtained by using the Doppler frequency.

If an angle between a velocity direction of each point and an incident direction of an ultrasound signal transmitted to an object is 0°, a velocity magnitude of the point may be accurately measured. However, a velocity magnitude of a point that is moving in another direction may not be accurately measured. In particular, if an angle between a velocity direction of each point and an incident direction of an ultrasound signal transmitted to an object is 90°, since a Doppler frequency is 0 Hz, a velocity magnitude of the point is measured as 0.

FIG. 2 is a block diagram of an ultrasound apparatus 100 according to an exemplary embodiment.

Referring to FIG. 2, the ultrasound apparatus 100 according to the exemplary embodiment includes a probe 110, an extraction unit 120, a vector information obtaining unit 130, and a vector information determination unit 140 (also referred to as vector information determiner). The extraction unit 120, the vector information obtaining unit 130, and the vector information determination unit 140 may be formed as microprocessors, although are not limited thereto.

The probe 110 includes a plurality of piezoelectric elements, and transmits an ultrasound signal to a predetermined point P (22) of an object and receives at least three response signals reflected from the predetermined point P (22). Each of the at least three response signals may have a Doppler frequency and a reception angle that is an incident angle from the probe 110.

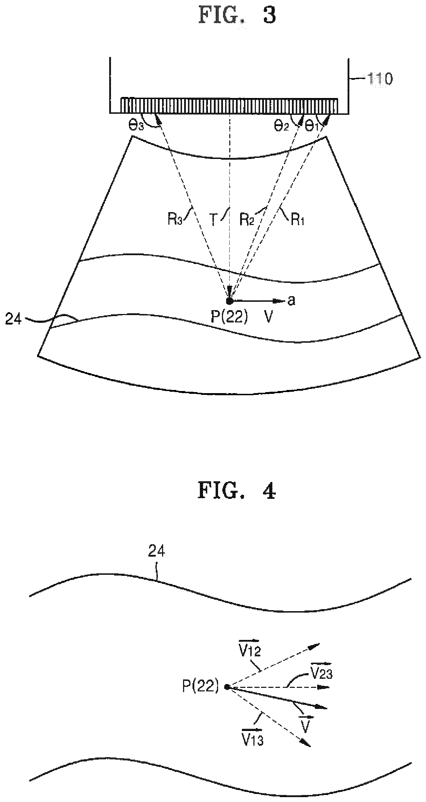

FIG. 3 is a schematic diagram showing an ultrasound signal T being transmitted to a predetermined point P (22) of an object and response signals $R_1$, $R_2$, and $R_3$ being received from the predetermined point P (22), using the ultrasound apparatus 100 illustrated in FIG. 2. Referring to FIG. 3, the probe 110 transmits the ultrasound signal T to the predetermined point P (22) in a blood vessel 24 of the object and receives the response signals $R_1$, $R_2$, and $R_3$ reflected from the predetermined point P (22). The response signals $R_1$, $R_2$, and $R_3$ respectively have reception angles $\theta_1$, $\theta_2$, and $\theta_3$.

The probe 110 may transmit the ultrasound signal T to the predetermined point P (22) of the object a plurality of times and may receive each of the at least three response signals reflected from the predetermined point P (22) a plurality of times. For example, if the three response signals include the response signals $R_1$, $R_2$, and $R_3$, the probe 110 receives each of the response signals $R_1$, $R_2$, and $R_3$ a plurality of times. A plurality of response signals $R_1$, a plurality of response signals $R_2$, and a plurality of response signals $R_3$ are referred to as ensemble signals. That is, ensemble signals refer to a plurality of response signals received at the same reception angle. By receiving a response signal a plurality of times, the reliability of data obtained by using the response signal may be improved.

The extraction unit 120 extracts a plurality of response signal pairs by combining the at least three response signals received by the probe 110 two-by-two. If n response signals are received, a certain number of response signal pairs may be extracted.. The certain number may be $_nC_2$ ($_nC_2 = \{n*(n-1)\} / (2*1)$).

For example, if the probe 110 receives the response signals $R_1$, $R_2$, and $R_3$, a response signal pair $R_{12}$ may be extracted by combining the response signals $R_1$ and $R_2$, a response signal pair $R_{13}$ may be extracted by combining the response signals $R_1$ and $R_3$, and a response signal pair $R_{23}$ may be extracted by combining the response signals $R_2$ and $R_3$.

The vector information obtaining unit 130 obtains a plurality of pieces of prediction vector information indicating a prediction velocity magnitude and a prediction velocity direction of the predetermined point P (22), based on reception angles and Doppler frequencies of response signals included in the plurality of response signal pairs. The vector information obtaining unit 130 may obtain a Doppler frequency of each response signal included in the plurality of response signal pairs by using autocorrelation between ensemble signals of the response signal.

Many different methods of obtaining vector information indicating a velocity magnitude and a velocity direction of a predetermined point by using Doppler frequencies and reception angles of two response signals may be used according to exemplary embodiments, and a detailed description thereof is not provided here.

The vector information determination unit 140 determines vector information indicating a velocity magnitude and a velocity direction of the predetermined point P (22) of the object, by using the plurality of pieces of prediction vector information.

In a conventional ultrasound apparatus using a vector Doppler technique, vector information of a predetermined point of an object is obtained by using only two response signals reflected from the predetermined point. However, if an angle between a reflection direction of any one response signal and a velocity direction of the predetermined point is close to 90°, accurate vector information may not be obtained. On the other hand, by using the ultrasound apparatus 100 according to exemplary embodiments or by using a method of generating an ultrasound image according to exemplary embodiments, since vector information of the predetermined point P (22) of the object is obtained by using at least three response signals reflected from the predetermined point P (22), accurate vector information may be obtained.

FIG. 4 is a schematic diagram for describing a method of obtaining vector information V of the predetermined point P (22) of the object, using the ultrasound apparatus 100 illustrated in FIG. 2.

FIG. 4 shows three pieces of prediction vector information $V_{12}$, $V_{23}$, and $V_{13}$ obtained by using response signal pairs of three response signals reflected from the predetermined point P (22) in the blood vessel 24.

The vector information determination unit 140 may obtain vector information V of the predetermined point P (22) by using a linear vector sum of the pieces of prediction vector information $V_{12}$, $V_{23}$, and $V_{13}$.

That is, the vector information determination unit 140 may obtain the vector information V of the predetermined point P (22) by using Equation 1.

[Equation 1]

$$V = aV_{12} + bV_{23} + cV_{13} \text{ (a, b, and c are real numbers)}$$

If all of a, b, and c are set as 1, the vector information V may be a simple vector sum of the pieces of prediction vector information $V_{12}$, $V_{23}$, and $V_{13}$. If all of a, b, and c are set as 1/3, the vector information V may be an average of the pieces of prediction vector information $V_{12}$, $V_{23}$, and $V_{13}$. Alternatively, a, b, and c may be set as different real numbers to apply a weight to a certain piece of prediction vector information.

A prediction velocity direction included in each piece of prediction vector information is not significantly different from an actual velocity direction of the predetermined point P (22), and a significant difference does not exist between prediction velocity directions.

Accordingly, the vector information determination unit 140 may determine any one of the prediction velocity directions included in the pieces of prediction vector information as being the velocity direction of the predetermined point P (22), and may determine a linear sum of prediction velocity magnitudes included in the pieces of prediction vector information as being the velocity magnitude of the predetermined point P (22). The velocity magnitude of the predetermined point P (22) may be obtained by using Equation 2. According to this technique, the amount of calculations for obtaining the vector information of the predetermined point P (22) may be reduced.

[Equation 2]

$$|V| = a|V_{12}| + b|V_{23}| + c|V_{13}| \text{ (a, b, and c are arbitrary real numbers)}$$

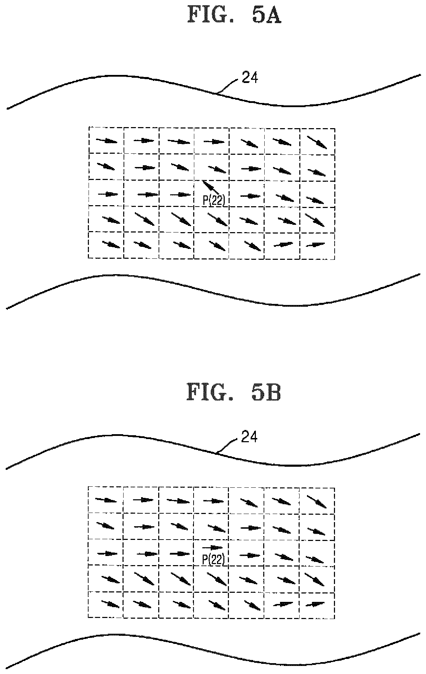

FIG. 5A is a schematic diagram showing vector information of a plurality of points including the predetermined point P (22), obtained using the ultrasound apparatus illustrated in FIG. 2. FIG. 5B is a schematic diagram showing a result of applying a smoothing filter to the vector information illustrated in FIG. 5A.

If a response signal reflected from the predetermined point P (22) has noise, vector information of the predetermined point P (22) may not be accurately obtained. Since the predetermined point P (22) of the object and peripheral points of the predetermined point P (22) have similar velocity magnitudes and velocity directions, accurate vector information may be obtained in consideration of the velocity magnitudes and the velocity directions of the peripheral points as well as the velocity magnitude and the velocity direction of the predetermined point P (22).

The vector information determination unit 140 determines vector information of each of a plurality of points located within a preset distance from the predetermined point P (22). In more detail, the probe 110 transmits an ultrasound signal to the plurality of points and receives at least three response signals reflected from each of the plurality of points.

The extraction unit 120 extracts a plurality of response signal pairs with respect to each of the plurality of points, and the vector information obtaining unit 130 obtains a plurality of pieces of prediction vector information of each of the plurality of points by using the plurality of response signal pairs. The vector information determination unit 140 determines vector information of the plurality of points by using the plurality of pieces of prediction vector information. The vector information of the plurality of points is represented by arrows in FIG. 5A. It is understood that the vector information of the plurality of points is not limited to being represented by the arrows shown in FIG. 5A, and may be represented in other ways as well.

A smoothing filter is commonly used in the fields of, for example, statistics and image processing, and is a technology for smoothing a data set to extract a pattern of the data set and to remove noise. For example, the smoothing filter may apply different weights to data of a plurality of points including a certain point so as to calculate their average and may determine the calculated average as data of the certain point.

FIG. 5B shows a result of applying a smoothing filter to the velocity magnitudes and the velocity directions of the plurality of points illustrated in FIG. 5A. As shown in FIG. 5B, by using the smoothing filter, the velocity magnitude and the velocity direction of the predetermined point P (22), which were significantly different from the velocity magnitudes and the velocity directions of the peripheral points before using the smoothing filter (as shown in FIG. 5A), are compensated.

The vector information determination unit 140 may compensate for only the velocity direction of the predetermined point P (22) by applying a smoothing filter to velocity directions of the plurality of points, or may compensate for only the velocity magnitude of the predetermined point P (22) by applying a smoothing filter to velocity magnitudes of the plurality of points.

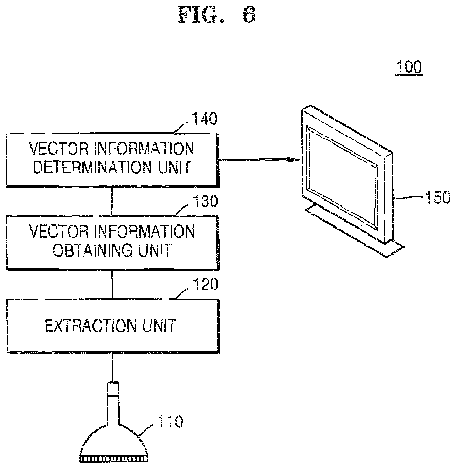

FIG. 6 is a block diagram of an ultrasound apparatus 100 according to another exemplary embodiment of the present invention. The ultrasound apparatus 100 according to the exemplary embodiment shown in FIG. 6 may further include a display unit 150.

The ultrasound apparatus 100 includes the display unit 150 which may display a color Doppler image of an object to a user. The display unit 150 may display the color Doppler image of the object by mapping velocity magnitudes of a plurality of points of the object to a color scale. It is understood that the display unit 150 is not required to display a color image, and may also display other types of images, e.g., black and white images, according to other exemplary embodiments.

Also, the ultrasound apparatus 100 may obtain vector information of a predetermined point of the object by using a method of obtaining the vector information of the predetermined point, and may display the color Doppler image of the object by mapping a velocity magnitude of the predetermined point to a color scale.

In more detail, the probe 110 transmits an ultrasound signal to a predetermined point included in an object and receives a response signal reflected from the predetermined point. The vector information determination unit 140 determines vector information indicating a velocity magnitude and a velocity direction of the predetermined point, based on a reception angle and a Doppler frequency of the response signal. The vector information determination unit 140 determines the vector information of the predetermined point based on reception angles and Doppler frequencies of at least three response signals. The display unit 150 may display a color Doppler image of the object by mapping the velocity magnitude of the predetermined point to a color scale.

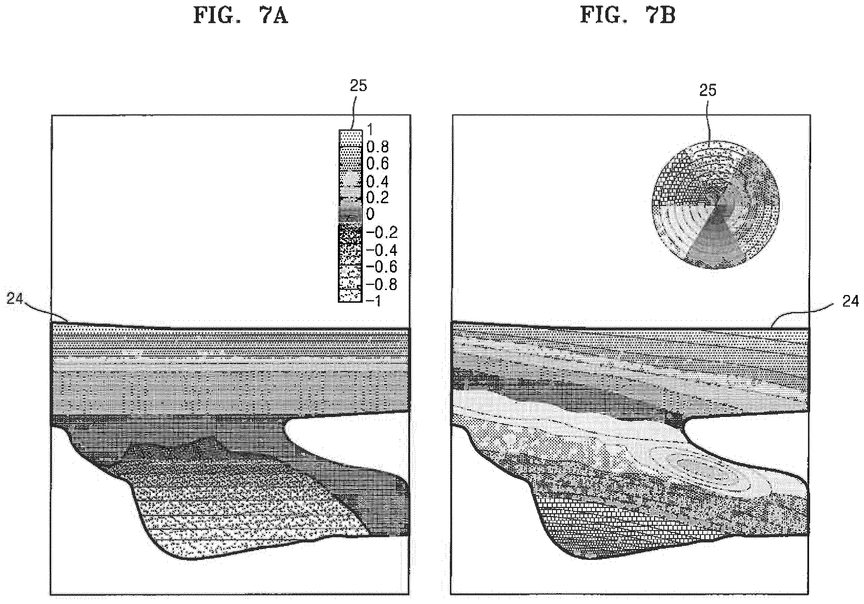

FIGS. 7A and 7B are color Doppler images in which vector information of a plurality of points of an object is mapped to a color scale 25.

The display unit 150 may display the color Doppler image of the object by mapping velocity magnitudes of the plurality of points to predetermined colors of the color scale 25. As such, a user may instinctively and easily check movement of, for example, blood in a blood vessel.

If only velocity magnitudes of the plurality of points are mapped to the color scale 25, two points having opposite velocity directions but having the same velocity magnitude may be mapped to the same color. Accordingly, the color Doppler image should be formed in consideration of velocity magnitudes and velocity directions of the plurality of points.

From among the plurality of points, the display unit 150 of the ultrasound apparatus 100 may map a velocity magnitude of a point having a first velocity direction component to a first color, and may map a velocity magnitude of a point having a second velocity direction component opposite to the first velocity direction component to a second color, as illustrated in FIG. 7A. The color scale 25 of FIG. 7A include colors corresponding to velocity magnitudes from 0 to -1, or from 0 to 1. That is, points having a velocity direction ① are mapped to the first color (corresponding to a part above the velocity magnitude 0 in the color scale 25 of FIG. 7A), and points having a velocity direction ② are mapped to the second color (corresponding to a part below the velocity magnitude 0 in the color scale 25 of FIG. 7A). As such, a user may easily check blood flow and may easily identify different types of blood flow, such as backflow.

The vector information determination unit 140 may estimate an overall velocity direction of the object by using the velocity directions of the plurality of points. For example, the vector information determination unit 140 may estimate an overall velocity direction of the object by using a vector sum of the vector information of the plurality of points.

The display unit 150 may display the color Doppler image of the object by mapping angle differences between the velocity directions of the plurality of points and the overall velocity direction of the object to the color scale 25. As such, a user may easily check blood flow and may easily identify different types of blood flow, such as turbulent flow or backflow.

The display unit 150 may display the color Doppler image of the object by mapping velocity magnitudes of the plurality of points and angle differences between the velocity directions of the plurality of points and the overall velocity direction of the object to the color scale 25, as illustrated in FIG. 7B. That is, referring to FIG. 7B, points having different velocity magnitudes and velocity directions are mapped to different colors. The circular color scale 25 of FIG. 7B includes colors corresponding to increasing or decreasing velocity magnitudes as indicated by the relative positions of the colors from a center of the circular color scale 25 toward a circumference of the circular color scale 25, and includes colors corresponding to angle differences between the velocity directions of the plurality of points and the overall velocity direction of the object as indicated by the relative angular positions of the colors within the circular color scale 25.

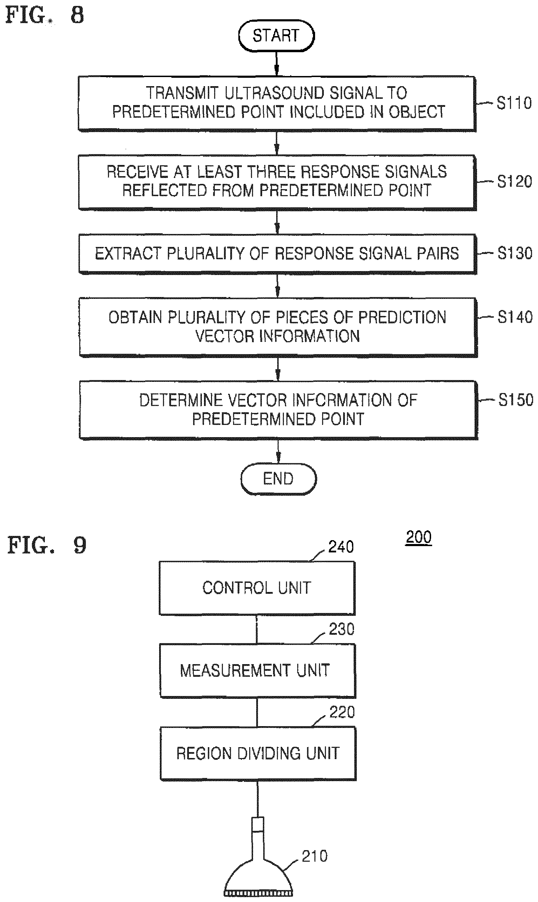

FIG. 8 is a flowchart of a method of generating an ultrasound image, according to an exemplary embodiment. Referring to FIG. 8, the method according to the exemplary embodiment includes operations performed in series by the ultrasound apparatus 100 illustrated in FIG. 2. Accordingly, although not mentioned particularly below, the descriptions provided above in relation to the ultrasound apparatus 100 illustrated in FIG. 2 may also be applied to the method illustrated in FIG. 8. It is understood that the method shown in FIG. 8 is not required to be performed by the ultrasound apparatus 100 illustrated in FIG. 2, and may alternatively be performed by other apparatuses according to other exemplary embodiments.

In operation S110, the ultrasound apparatus 100 transmits an ultrasound signal to the predetermined point P (22) included in the object.

In operation S120, the ultrasound apparatus 100 receives at least three response signals reflected from the predetermined point P (22). Each of the at least three response signals has a Doppler frequency and a reception angle.

In operation S130, the ultrasound apparatus 100 extracts a plurality of response signal pairs by combining the at least three response signals two-by-two. If the ultrasound apparatus 100 receives n response signals, a certain number of response signal pairs may be extracted.. The certain number may be $_nC_2$ ($_nC_2 = \{n*(n-1)\} / (2*1)$).

In operation S140, the ultrasound apparatus 100 obtains a plurality of pieces of prediction vector information by using the plurality of response signal pairs. The plurality of pieces of prediction vector information includes a prediction velocity magnitude and a prediction velocity direction of the predetermined point P (22).

In operation S150, the ultrasound apparatus 100 determines vector information indicating a velocity magnitude and a velocity direction of the predetermined point P (22) by using the plurality of pieces of prediction vector information.

FIG. 9 is a block diagram of an ultrasound apparatus 200 according to another exemplary embodiment.

The ultrasound apparatus 200 according to the exemplary embodiment shown in FIG. 9 locates a region of interest in an image generated by a color Doppler apparatus and a range gate in an image generated by a spectral Doppler apparatus accurately on a region of an object to be measured, and adjusts a steering angle of an ultrasound signal in such a way that an angle between a velocity direction of the region to be measured and an incident direction of the ultrasound signal transmitted to the object is not 90°.

Referring to FIG. 9, the ultrasound apparatus 200 according to the exemplary embodiment includes a probe 210, a region dividing unit 220, a measurement unit 230, and a control unit 240. The region dividing unit 220 and the control unit 240 may be formed as microprocessors, although are not limited thereto.

Initially, the region dividing unit 220 divides the object into a plurality of regions. The region dividing unit 220 may divide only a portion of the object into a plurality of regions.

The probe 210 transmits an ultrasound signal to the plurality of regions and receives response signals reflected from the plurality of regions.

The measurement unit 230 measures power levels or Doppler frequencies of the received response signals.

The control unit 240 determines a monitoring region from among the plurality of regions by using the power levels or the Doppler frequencies of the response signals.

When a user desires to measure movement of blood in a blood vessel, the region of interest should be adjusted to include blood in the blood vessel and the range gate should be adjusted to be located in blood in the blood vessel. However, if the user manually adjusts the region of interest and the range gate, an inaccurate Doppler image may be generated.

Accordingly, when a user desires to measure movement of blood in a blood vessel, the ultrasound apparatus 200 automatically adjusts the monitoring region of the ultrasound apparatus 200 to be located in blood in the blood vessel and correspondingly adjusts the region of interest or the range gate.

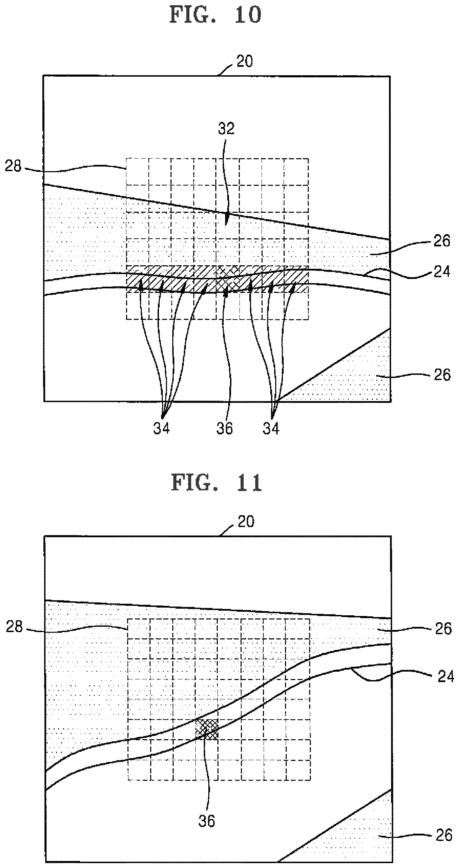

FIGS. 10 and 11 are schematic diagrams for describing methods of determining a monitoring region 36 from among a plurality of regions 28 of an object 20, using the ultrasound apparatus 200 illustrated in FIG. 9. According to this example, a user desires to monitor movement of blood in a blood vessel 24. It is understood that many other types of objects and flows may also be monitored.

FIGS. 10 and 11 show the object 20 including tissues 26 and the blood vessel 24, and a portion of the object 20 is divided into the plurality of regions 28.

In FIG. 10, the control unit 240 arbitrarily sets an initial region 32 from among the plurality of regions 28.

The measurement unit 230 measures power levels or Doppler frequencies of response signals reflected from the plurality of regions 28.

In general, power levels of response signals reflected from the tissues 26 are higher than those of response signals reflected from blood, and a Doppler frequency of a response signal reflected from the tissue 26 is less than that of a response signal reflected from blood.

The control unit 240 selects regions 34 reflecting response signals having Doppler frequencies in a first predetermined range and determines one of the regions 34, which is the closest to the initial region 32 set by the control unit 240, as the monitoring region 36. The first predetermined range may be a Doppler frequency range equal to or greater than a preset value and equal to or less than the highest Doppler frequency from among the Doppler frequencies of the response signals. Since the regions 34 reflecting response signals having Doppler frequencies greater than the preset value are mostly distributed in a region where blood flows in the blood vessel 24, the control unit 240 determines one of the regions 34, which is the closest to the initial region 32, as the monitoring region 36. However, the first predetermined range may vary within a range that is well known to one of ordinary skill in the art.

Referring to FIG. 11, the control unit 240 may not set an initial region and may determine a region reflecting a response signal having a Doppler frequency in a second predetermined range from among the Doppler frequencies of the received response signals, as the monitoring region 36. In this case, the second predetermined range may include the highest Doppler frequency from among the Doppler frequencies of the response signals, and may vary within a range that is well known to one of ordinary skill in the art.

Also, the control unit 240 may select regions 34 reflecting response signals having a power level in a third predetermined range and may determine one of the regions 34, which is the closest to the initial region 32 set by the control unit 240, as the monitoring region 36. The third predetermined range may be a power level range greater than 0 and equal to or less than a preset value. Since the regions 34 reflecting response signals having a power level less than the preset value are mostly distributed in a region where blood flows in the blood vessel 24, the control unit 240 determines one of the regions 34, which is the closest to the initial region 32, as the monitoring region 36. However, the third predetermined range may vary within a range that is well known to one of ordinary skill in the art.

Furthermore, as illustrated in FIG. 11, the control unit 240 may not set an initial region and may determine a region reflecting a response signal having a power level in a fourth predetermined range, as the monitoring region 36. In this case, the fourth predetermined range may include the lowest power level from among the power levels of the response signals, and may vary within a range that is well known to one of ordinary skill in the art.

As described above, the response signals reflected from the plurality of regions 28 include the response signals reflected from the tissues 26 and the response signals reflected from blood. The response signals reflected from the tissues 26 may be removed by applying a clutter filter to the received response signals. As such, the response signals reflected from the tissues 26, which have power levels relatively higher than those of response signals reflected from blood, may be removed. In this case, the control unit 240 may determine a region reflecting a response signal having a power level in a fifth predetermined range from among the response signals that pass through the clutter filter, as the monitoring region 36. The fifth predetermined range may include the highest power level from among the power levels of the response signals that pass through the clutter filter, and may vary within a range that is well known to one of ordinary skill in the art.

In the color Doppler apparatus, the control unit 240 may adjust the region of interest in such a way that the monitoring region 36 is included in the region of interest. In the spectral Doppler apparatus, the control unit 240 may adjust the range gate in such a way that the range gate is located in the monitoring region 36.

Since the monitoring region 36 is determined based on power levels or Doppler frequencies of response signals, a user may automatically obtain accurate location information of blood and may obtain an accurate Doppler image.

Although a region corresponding to blood in a blood vessel is determined as a monitoring region in the above description, it will be understood by one of ordinary skill in the art that, due to a simple modification, a region corresponding to tissues or other objects may also be determined as the monitoring region.

As described above, if an angle between an incident direction of an ultrasound signal transmitted to an object and a velocity direction of the predetermined point P (22) is close to 90°, a velocity magnitude of the predetermined point P (22) may not be easily measured in an accurate fashion. Accordingly, a steering angle of the ultrasound signal should be adjusted.

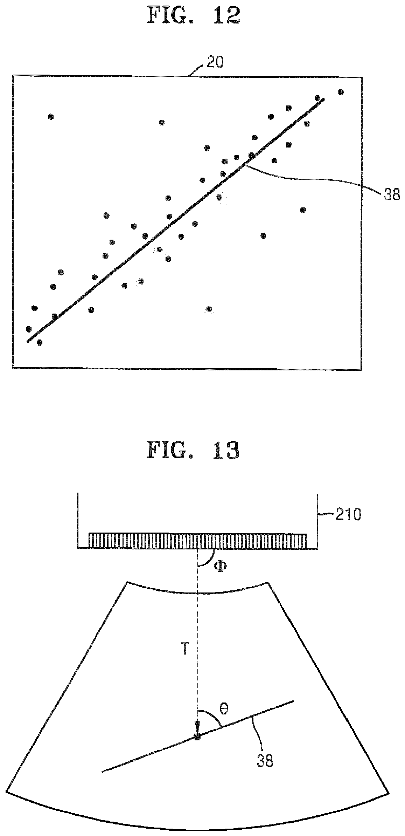

FIG. 12 is a schematic diagram for describing a method of determining a location of the blood vessel 24 included in the object 20, using the ultrasound apparatus 200 illustrated in FIG. 9.

The measurement unit 230 measures power levels or Doppler frequencies of response signals reflected from a plurality of regions located within a preset distance from the monitoring region 36.

Since Doppler frequencies of response signals reflected from blood are higher than those of response signals reflected from the tissues 26, if a high-pass filter is applied to the response signals, the response signals that pass through the high-pass filter are mostly distributed along the blood vessel 24 and are scarcely distributed in the other regions.

The control unit 240 obtains a modeled line 38 by applying a fitting method to the Doppler frequencies of the response signals. The fitting method may include many different type of fitting, e.g., linear fitting or curve fitting, and the modeled line 38 may include a linear line or a curved line. The fitting method is a method of obtaining a line indicating a tendency of data distribution, and is used in statistics.

A location of the modeled line 38 obtained by applying the fitting method to the Doppler frequencies of the response signals very closely corresponds to the location of the blood vessel 24. Since blood in the blood vessel 24 flows along the blood vessel 24, the control unit 240 adjusts a steering angle of an ultrasound signal transmitted to the plurality of regions in such a way that an angle between the obtained modeled line 38 and an incident direction of the ultrasound signal is a predetermined angle. If the modeled line 38 is a curved line, a direction of a tangent to the curved line may be determined as a direction of the blood vessel 24. Alternatively, the control unit 240 may apply the fitting method to velocity magnitudes of the plurality of regions, which are obtained from the Doppler frequencies of the response signals, based on the concept that blood has velocity magnitudes less than those of the tissues 26.

Also, the control unit 240 may obtain the modeled line 38 by applying the fitting method to power levels of the response signals. Since power levels of response signals reflected from blood are lower than those of response signals reflected from the tissues 26, the control unit 240 may obtain reciprocals of the power levels of the response signals and may apply a high-pass filter to the reciprocals of the power levels so as to reduce a data distribution of the response signals reflected from the tissues 26. As such, data of the response signals reflected from blood are mostly distributed along the blood vessel 24, and data of the response signals reflected from regions other than blood are reduced.

If the fitting method is applied to the reciprocals of the power levels, the modeled line 38 illustrated in FIG. 12 may be obtained, and the control unit 240 may adjust the steering angle of the ultrasound signal transmitted to the plurality of regions in such a way that the angle between the obtained modeled line 38 and the incident direction of the ultrasound signal is the predetermined angle.

FIG. 13 is a schematic diagram for describing a method of adjusting a steering angle of an ultrasound signal, using the ultrasound apparatus 200 illustrated in FIG. 9.

Referring to FIG. 13, an angle between the ultrasound signal and the modeled line 38 is θ, and the steering angle of the ultrasound signal is Φ. The control unit 240 adjusts the steering angle Φ of the ultrasound signal transmitted to the object 20 in such a way that the angle θ between an incident direction of the ultrasound signal and the modeled line 38 is not 90°. That is, the control unit 240 may adjust the steering angle Φ in such a way that the angle θ between the incident direction of the transmitted ultrasound signal and the modeled line 38 is close to 0°.

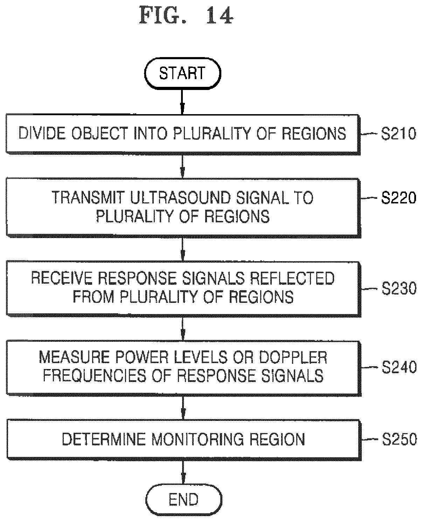

FIG. 14 is a flowchart of a method of generating an ultrasound image, according to another exemplary embodiment. Referring to FIG. 14, the method according to another exemplary embodiment includes operations performed in series by the ultrasound apparatus 200 illustrated in FIG. 9. Accordingly, although not mentioned particularly below, the descriptions provided above in relation to the ultrasound apparatus 200 illustrated in FIG. 9 may also be applied to the method illustrated in FIG. 14. It is understood that the method shown in FIG. 14 is not required to be performed by the ultrasound apparatus 200 illustrated in FIG. 9, and may alternatively be performed by other apparatuses according to other exemplary embodiments.

In operation S210, the ultrasound apparatus 200 divides an image of an object into a plurality of regions. The ultrasound apparatus 200 may divide a portion of the object into a plurality of regions.

In operation S220, the ultrasound apparatus 200 transmits an ultrasound signal to the plurality of regions.

In operation S230, the ultrasound apparatus 200 receives response signals reflected from the plurality of regions.

In operation S240, the ultrasound apparatus 200 measures power levels or Doppler frequencies of the response signals reflected from the plurality of regions.

In operation S250, the ultrasound apparatus 200 determines a monitoring region from among the plurality of regions by using the power levels or the Doppler frequencies of the response signals.

The ultrasound apparatus 200 may adjust a region of interest in such a way that the monitoring region is included in the region of interest, or may adjust a range gate in such a way that the range gate is located in the monitoring region.

Exemplary embodiments of the present disclosure can be written as computer programs and can be implemented in general-use digital computers that execute the programs using a computer-readable recording medium.

Examples of the computer-readable recording medium include magnetic storage media (e.g., ROM, floppy disks, hard disks, etc.), optical recording media (e.g., CD-ROMs or DVDs), etc.

**Claims**

**1.** A method of generating an ultrasound image of velocity, the method comprising:

transmitting an ultrasound signal (T) to a predetermined point (P(22)) included in an object (24) and receiving at least three response signals ($R_1$, $R_2$, $R_3$) reflected from the predetermined point (P(22)), each of the at least three response signals ($R_1$, $R_2$, $R_3$) respectively has a Doppler frequency and a reception angle ($\theta_1$, $\theta_2$, $\theta_3$);

extracting at least three response signal pairs ($R_{12}$, $R_{13}$, $R_{23}$) by combining each of the received at least three response signals ($R_1$, $R_2$, $R_3$) with each of the other signals ($R_1$, $R_2$, $R_3$);

obtaining at least three pieces of prediction vector information ($V_{12}$, $V_{13}$, $V_{23}$), each of the at least three pieces of prediction vector information ($V_{12}$, $V_{13}$, $V_{23}$) indicating a prediction velocity magnitude and a prediction velocity direction of the predetermined point, based on reception angles and Doppler frequencies of the response signals included in the at least three response signal pairs ($R_{12}$, $R_{13}$, $R_{23}$); and

determining two-dimensional vector information (V) indicating a velocity magnitude and a velocity direction of the predetermined point, based on the at least three pieces of prediction vector information ($V_{12}$, $V_{13}$, $V_{23}$).

2. The method of claim 1, wherein the determining of the vector information (V) comprises determining the vector information (V) based on a linear vector sum of the at least three pieces of prediction vector information ($V_{12}$, $V_{13}$, $V_{23}$).

3. The method of claim 1, wherein the determining of the vector information (V) comprises:

determining any one of the plurality of prediction velocity directions included in the at least three pieces of prediction vector information ($V_{12}$, $V_{13}$, $V_{23}$), as the velocity direction of the predetermined point; and

determining a linear sum of the plurality of prediction velocity magnitudes included in the at least three pieces of prediction vector information ($V_{12}$, $V_{13}$, $V_{23}$), as the velocity magnitude of the predetermined point.

4. The method of claim 1, further comprising determining vector information (V) of a plurality of additional points located within a preset distance from the predetermined point.

5. The method of claim 4, wherein the determining of the vector information (V) comprises:

compensating for the velocity direction of the predetermined point by applying a smoothing filter to velocity directions of the plurality of additional points; and

determining the compensated velocity direction as the velocity direction of the predetermined point.

6. The method of claim 4, wherein the determining of the vector information (V) comprises:

compensating for the velocity magnitude of the predetermined point by applying a smoothing filter to velocity magnitudes of the plurality of additional points; and

determining the compensated velocity magnitude as the velocity magnitude of the predetermined point.

7. The method of claim 1, wherein:

the transmitting of the ultrasound signal (T) comprises transmitting the ultrasound signal (T) a plurality of times and receiving each of the at least three response signals ($R_1$, $R_2$, $R_3$) a plurality of times, a plurality of the same received response signal forming ensemble signals for the respective response signal, and

the obtaining of the at least three pieces of prediction vector information ($V_{12}$, $V_{13}$, $V_{23}$) comprises obtaining a Doppler frequency of each response signal (R) included in the plurality of response signal pairs ($R_{12}$, $R_{13}$, $R_{23}$) by using autocorrelation between the ensemble signals of the response signals.

8. An ultrasound apparatus for velocity imaging (100) comprising:

a probe (110) which is adapted to transmit an ultrasound signal (T) to a predetermined point (P(22)) included in an object and to receive at least three response signals ($R_1$, $R_2$, $R_3$) reflected from the predetermined point (P(22)); wherein each of the at least three response signals ($R_1$, $R_2$, $R_3$) respectively has a Doppler frequency and a reception angle ($\theta_1$, $\theta_2$, $\theta_3$);

an extraction unit (120) which is adapted to extract a plurality of response signal pairs ($R_{12}$, $R_{13}$, $R_{23}$) by combining each of the received at least three response signals ($R_1$, $R_2$, $R_3$) with each of the other signals ($R_1$, $R_2$, $R_3$);

a vector information obtaining unit (130) which is adapted to obtain at least three pieces of prediction vector information ($V_{12}$, $V_{13}$, $V_{23}$), each of the at least three pieces of prediction vector information ($V_{12}$, $V_{13}$, $V_{23}$) indicating a prediction velocity magnitude and a prediction velocity direction of the predetermined point (P(22)),

based on reception angles ($\theta_1$, $\theta_2$, $\theta_3$) and Doppler frequencies of response signals included in the at least three of the response signal pairs ($R_{12}$, $R_{13}$, $R_{23}$); and

a vector information determination unit (140) which determines two-dimensional vector information (V) indicating a velocity magnitude and a velocity direction of the predetermined point, based on the at least three pieces of prediction vector information ($V_{12}$, $V_{13}$, $V_{23}$).

9. The ultrasound apparatus of claim 8, wherein the vector information determination unit (140) is adapted to determine the vector information (V) by using a linear vector sum of the at least three pieces of prediction vector information ($V_{12}$, $V_{13}$, $V_{23}$).

10. The ultrasound apparatus of claim 8, wherein the vector information determination unit (140) is adapted to determine any one of the plurality of prediction velocity directions included in the at least three pieces of prediction vector information ($V_{12}$, $V_{13}$, $V_{23}$), as the velocity direction of the predetermined point (P(22)), and to determine a linear sum of the plurality of prediction velocity magnitudes included in the at least three pieces of prediction vector information ($V_{12}$, $V_{13}$, $V_{23}$), as the velocity magnitude of the predetermined point (P(22)).

11. The ultrasound apparatus of claim 8, wherein the vector information determination unit (140) is adapted to determine vector information (V) of a plurality of additional points located within a preset distance from the predetermined point (P(22)).

12. The ultrasound apparatus of claim 11, wherein the vector information determination unit (140) is adapted to compensate for the velocity direction of the predetermined point (P(22)) by applying a smoothing filter to velocity directions of the plurality of additional points, and to determine the compensated velocity direction as the velocity direction of the predetermined point (P(22)).

13. The ultrasound apparatus of claim 11, wherein the vector information determination unit (140) is adapted to compensate for the velocity magnitude of the predetermined point (P(22)) by applying a smoothing filter to velocity magnitudes of the plurality of additional points, and to determine the compensated velocity magnitude as the velocity magnitude of the predetermined point (P(22)).

14. The ultrasound apparatus of claim 8, wherein the probe (110) is adapted to transmit the ultrasound signal (T) a plurality of times and to receive each of the at least three response signals ($R_1$, $R_2$, $R_3$) a plurality of times, a plurality of the same received response signal (R) forming ensemble signals for the respective response signal (R), and the vector information obtaining unit (140) is adapted to obtain a Doppler frequency of each response signal (R) included in the plurality of response signal pairs ($R_{12}$, $R_{13}$, $R_{23}$) by using autocorrelation between the ensemble signals of the response signal (R).

15. A non-transitory computer-readable recording medium having recorded thereon a computer program for executing the method of any one of claims 1 through 7.

**Patentansprüche**

1. Verfahren zum Erzeugen eines Ultraschallbildes der Geschwindigkeit, wobei das Verfahren umfasst:

Übermitteln eines Ultraschallsignals (T) an einen vorbestimmten Punkt (P(22)), der in ein Objekt (24) eingeschlossen ist, und Empfangen von mindestens drei Antwortsignalen ($R_1$, $R_2$, $R_3$), die von dem vorbestimmten Punkt (P(22)) reflektiert wurden,
wobei jedes der drei Antwortsignale ($R_1$, $R_2$, $R_3$) jeweils eine Dopplerfrequenz und einen Empfangswinkel ($\theta_1$, $\theta_2$, $\theta_3$) aufweist;
Extrahieren von mindestens drei Antwortsignalpaaren ($R_{12}$, $R_{13}$, $R_{23}$) durch Kombinieren von jedem der empfangenen mindestens drei Antwortsignale ($R_1$, $R_2$, $R_3$) mit jedem der anderen Signale ($R_1$, $R_2$, $R_3$);
Erhalten von mindestens drei Teilen von Vorhersagevektorinformationen ($V_{12}$, $V_{13}$, $V_{23}$), wobei jedes der mindestens drei Teile der Vorhersagevektorinformationen ($V_{12}$, $V_{13}$, $V_{23}$) einen Vorhersagegeschwindigkeitsbetrag und eine Vorhersagegeschwindigkeitsrichtung des vorbestimmten Punkts basierend auf Empfangswinkeln und Dopplerfrequenzen der Antwortsignale angibt, die in die mindestens drei Antwortsignalpaare ($R_{12}$, $R_{13}$, $R_{23}$) eingeschlossen sind; und
Bestimmen von zweidimensionalen Vektorinformationen (V), die einen Geschwindigkeitsbetrag und eine Geschwindigkeitsrichtung des vorbestimmten Punkts angeben, basierend auf den mindestens drei Teilen der Vorhersagevektorinformationen ($V_{12}$, $V_{13}$, $V_{23}$).

2. Verfahren nach Anspruch 1, wobei das Bestimmen der Vektorinformationen (V) Bestimmen der Vektorinformationen (V) basierend auf einer linearen Vektorsumme der mindestens drei Teile der Vorhersagevektorinformationen ($V_{12}$, $V_{13}$, $V_{23}$) umfasst.

3. Verfahren nach Anspruch 1, wobei das Bestimmen der Vektorinformationen (V) umfasst:

Bestimmen von einer beliebigen von der Vielzahl der Vorhersagegeschwindigkeitsrichtungen, die in die mindestens drei Teile der Vorhersagevektorinformationen ($V_{12}$, $V_{13}$, $V_{23}$) eingeschlossen sind, als Geschwindigkeitsrichtung des vorbestimmten Punkts; und
Bestimmen einer linearen Summe von der Vielzahl der Vorhersagegeschwindigkeitsbeträge, die in die mindestens drei Teile der Vorhersagevektorinformationen ($V_{12}$, $V_{13}$, $V_{23}$) eingeschlossen sind, als Geschwindigkeitsbetrag des vorbestimmten Punkts.

4. Verfahren nach Anspruch 1, des Weiteren umfassend Bestimmen von Vektorinformationen (V) von einer Vielzahl von zusätzlichen Punkten, die sich innerhalb einer vorgegebenen Distanz zu dem vorbestimmten Punkt befinden.

5. Verfahren nach Anspruch 4, wobei das Bestimmen der Vektorinformationen (V) umfasst:

Kompensieren der Geschwindigkeitsrichtung des vorbestimmten Punkts durch Anwenden eines Glättungsfilters auf Geschwindigkeitsrichtungen von der Vielzahl der zusätzlichen Punkte; und
Bestimmen der kompensierten Geschwindigkeitsrichtung als Geschwindigkeitsrichtung des vorbestimmten Punkts.

6. Verfahren nach Anspruch 4, wobei das Bestimmen der Vektorinformationen (V) umfasst:

Kompensieren des Geschwindigkeitsbetrags des vorbestimmten Punkts durch Anwenden eines Glättungsfilters auf Geschwindigkeitsbeträge von der Vielzahl der zusätzlichen Punkte; und
Bestimmen des kompensierten Geschwindigkeitsbetrags als Geschwindigkeitsbetrag des vorbestimmten Punkts.

7. Verfahren nach Anspruch 1, wobei:

das Übermitteln des Ultraschallsignals (T) mehrmaliges Übermitteln des Ultraschallsignals (T) und mehrmaliges Empfangen von jedem von den mindestens drei Antwortsignalen ($R_1$, $R_2$, $R_3$) umfasst, wobei eine Vielzahl desselben empfangenen Antwortsignals Ensemblesignale für das jeweilige Antwortsignal bildet, und
das Erhalten der mindestens drei Teile der Vorhersagevektorinformationen ($V_{12}$, $V_{13}$, $V_{23}$) Erhalten einer Dopplerfrequenz von jedem Antwortsignal (R) umfasst, das in die Vielzahl der Antwortsignalpaare ($R_{12}$, $R_{13}$, $R_{23}$) eingeschlossen ist, indem Autokorrelation zwischen den Ensemblesignalen der Antwortsignale verwendet wird.

8. Ultraschallsignal zur Geschwindigkeitsbildgebung (100), umfassend:

eine Sonde (110), die zum Übermitteln eines Ultraschallsignals (T) an einen vorbestimmten Punkt (P(22)), der in ein Objekt eingeschlossen ist, und Empfangen von mindestens drei Antwortsignalen ($R_1$, $R_2$, $R_3$) vorgesehen ist, die von dem vorbestimmten Punkt (P(22)) reflektiert wurden, wobei jedes der drei Antwortsignale ($R_1$, $R_2$, $R_3$) jeweils eine Dopplerfrequenz und einen Empfangswinkel ($\theta_1$, $\theta_2$, $\theta_3$) aufweist;
eine Extraktionseinheit (120), die zum Extrahieren von einer Vielzahl von Antwortsignalpaaren ($R_{12}$, $R_{13}$, $R_{23}$) durch Kombinieren von jedem der empfangenen mindestens drei Antwortsignale ($R_1$, $R_2$, $R_3$) mit jeweils den anderen Signalen ($R_1$, $R_2$, $R_3$) vorgesehen ist;
eine Vektorinformationen erhaltende Einheit (130), die zum Erhalten von mindestens drei Teilen von Vorhersagevektorinformationen ($V_{12}$, $V_{13}$, $V_{23}$) vorgesehen ist, wobei jedes der mindestens drei Teile der Vorhersagevektorinformationen ($V_{12}$, $V_{13}$, $V_{23}$) einen Vorhersagegeschwindigkeitsbetrag und eine Vorhersagegeschwindigkeitsrichtung des vorbestimmten Punkts (P(22)) basierend auf Empfangswinkeln ($\theta_1$, $\theta_2$, $\theta_3$) und Dopplerfrequenzen der Antwortsignale angibt, die in die mindestens drei Antwortsignalpaare ($R_{12}$, $R_{13}$, $R_{23}$) eingeschlossen sind; und
eine Vektorinformationen bestimmende Einheit (140), die zweidimensionale Vektorinformationen (V), die einen Geschwindigkeitsbetrag und eine Geschwindigkeitsrichtung des vorbestimmten Punkts angeben, basierend auf den mindestens drei Teilen der Vorhersagevektorinformationen ($V_{12}$, $V_{13}$, $V_{23}$) bestimmt.

9. Ultraschallvorrichtung nach Anspruch 8, wobei die Vektorinformationen bestimmende Einheit (140) zum Bestimmen der Vektorinformationen (V) durch Verwendung einer linearen Vektorsumme der mindestens drei Teile der Vorhersagevektorinformationen ($V_{12}$, $V_{13}$, $V_{23}$) vorgesehen ist.

10. Ultraschallvorrichtung nach Anspruch 8, wobei die Vektorinformationen bestimmende Einheit (140) zum Bestimmen von jeglichen von der Vielzahl der Vorhersagegeschwindigkeitsrichtungen, die in die mindestens drei Teile der Vorhersagevektorinformationen ($V_{12}$, $V_{13}$, $V_{23}$) eingeschlossen sind, als Geschwindigkeitsrichtung des vorbestimmten Punkts (P(22)), und zum Bestimmen einer linearen Summe von der Vielzahl der Vorhersagegeschwindigkeitsbeträge, die in die mindestens drei Teile der Vorhersagevektorinformationen ($V_{12}$, $V_{13}$, $V_{23}$) eingeschlossen sind, als Geschwindigkeitsbetrag des vorbestimmten Punkts (P(22)) vorgesehen ist.

11. Ultraschallvorrichtung nach Anspruch 8, wobei die Vektorinformationen bestimmende Einheit (140) zum Bestimmen von Vektorinformationen (V) von der Vielzahl der zusätzlichen Punkte vorgesehen ist, die sich innerhalb einer vorgegebenen Distanz zu dem vorbestimmten Punkt (P(22)) befinden.

12. Ultraschallvorrichtung nach Anspruch 11, wobei die Vektorinformationen bestimmende Einheit (140) zum Kompensieren der Geschwindigkeitsrichtung des vorbestimmten Punkts (P(22)), indem ein Glättungsfilter auf Geschwindigkeitsrichtungen von der Vielzahl der zusätzlichen Punkte angewendet wird, und zum Bestimmen der kompensierten Geschwindigkeitsrichtung als Geschwindigkeitsrichtung des vorbestimmten Punkts (P(22)) vorgesehen ist.

13. Ultraschallvorrichtung nach Anspruch 11, wobei die Vektorinformationen bestimmende Einheit (140) zum Kompensieren des Geschwindigkeitsbetrags des vorbestimmten Punkts (P(22)), indem ein Glättungsfilter auf Geschwindigkeitsbeträge von der Vielzahl der zusätzlichen Punkte angewendet wird, und zum Bestimmen des kompensierten Geschwindigkeitsbetrags als Geschwindigkeitsbetrag des vorbestimmten Punkts (P(22)) vorgesehen ist.

14. Ultraschallvorrichtung nach Anspruch 8, wobei die Sonde (110) zum mehrmaligen Übermitteln des Ultraschallsignals (T) und zum mehrmaligen Empfangen von jedem der mindestens drei Antwortsignale ($R_1$, $R_2$, $R_3$) vorgesehen ist, wobei eine Vielzahl desselben empfangenen Antwortsignals (R) Ensemblesignale für das jeweilige Antwortsignal (R) bildet, und die Vektorinformationen erhaltene Einheit (140) zum Erhalten einer Dopplerfrequenz von jedem Antwortsignal (R) vorgesehen ist, das in die Vielzahl der Antwortsignalpaare ($R_{12}$, $R_{13}$, $R_{23}$) eingeschlossen ist, indem Autokorrelation zwischen den Ensemblesignalen des Antwortsignals (R) verwendet wird.

15. Nicht-vorübergehendes computerlesbares Aufzeichnungsmedium, auf dem ein Computerprogramm zum Ausführen des Verfahrens nach einem der Ansprüche 1 bis 7 aufgezeichnet ist.

**Revendications**

1. Procédé de génération d'une image ultrasonore de vitesse, le procédé comprenant :

l'émission d'un signal ultrasonore (T) jusqu'à un point prédéterminé (P(22)) inclus dans un objet (24) et la réception d'au moins trois signaux de réponse ($R_1$, $R_2$, $R_3$) réfléchis depuis le point prédéterminé (P(22)), chacun des au moins trois signaux de réponse ($R_1$, $R_2$, $R_3$) ayant respectivement une fréquence Doppler et un angle de réception ($\theta_1$, $\theta_2$, $\theta_3$) ;
l'extraction d'au moins trois paires de signaux de réponse ($R_{12}$, $R_{13}$, $R_{23}$) par combinaison de chacun des au moins trois signaux de réponse reçus ($R_1$, $R_2$, $R_3$) avec chacun des autres signaux ($R_1$, $R_2$, $R_3$) ;
l'obtention d'au moins trois éléments d'information vectorielle de prédiction ($V_{12}$, $V_{13}$, $V_{23}$), chacun des au moins trois éléments d'information vectorielle de prédiction ($V_{12}$, $V_{13}$, $V_{23}$) indiquant une amplitude de vitesse de prédiction et une direction de vitesse de prédiction du point prédéterminé, sur la base d'angles de réception et de fréquences Doppler des signaux de réponse inclus dans les au moins trois paires de signaux de réponse ($R_{12}$, $R_{13}$, $R_{23}$) ; et
la détermination d'informations vectorielles bidimensionnelles (V) indiquant une amplitude de vitesse et une direction de vitesse du point prédéterminé, sur la base des au moins trois éléments d'information vectorielle de prédiction ($V_{12}$, $V_{13}$, $V_{23}$).

**2.** Procédé de la revendication 1, dans lequel la détermination des informations vectorielles (V) comprend la détermination des informations vectorielles (V) sur la base d'une somme vectorielle linéaire des au moins trois éléments d'information vectorielle de prédiction ($V_{12}$, $V_{13}$, $V_{23}$).

**3.** Procédé de la revendication 1, dans lequel la détermination des informations vectorielles (V) comprend :

la détermination de l'une quelconque de la pluralité de directions de vitesse de prédiction incluses dans les au moins trois éléments d'information vectorielle de prédiction ($V_{12}$, $V_{13}$, $V_{23}$) comme la direction de vitesse du point prédéterminé ; et
la détermination d'une somme linéaire de la pluralité d'amplitudes de vitesse de prédiction incluses dans les au moins trois éléments d'information vectorielle de prédiction ($V_{12}$, $V_{13}$, $V_{23}$) comme l'amplitude de vitesse du point prédéterminé.

**4.** Procédé de la revendication 1, comprenant en outre la détermination d'informations vectorielles (V) d'une pluralité de points supplémentaires situés à moins d'une distance prédéfinie du point prédéterminé.

**5.** Procédé de la revendication 4, dans lequel la détermination des informations vectorielles (V) comprend :

la compensation de la direction de vitesse du point prédéterminé par application d'un filtre de lissage à des directions de vitesse de la pluralité de points supplémentaires ; et
la détermination de la direction de vitesse compensée comme la direction de vitesse du point prédéterminé.

**6.** Procédé de la revendication 4, dans lequel la détermination des informations vectorielles (V) comprend :

la compensation de l'amplitude de vitesse du point prédéterminé par application d'un filtre de lissage à des amplitudes de vitesse de la pluralité de points supplémentaires ; et
la détermination de l'amplitude de vitesse compensée comme l'amplitude de vitesse du point prédéterminé.

**7.** Procédé de la revendication 1, dans lequel :

l'émission du signal ultrasonore (T) comprend l'émission du signal ultrasonore (T) une pluralité de fois et la réception de chacun des au moins trois signaux de réponse ($R_1$, $R_2$, $R_3$) une pluralité de fois, une pluralité du même signal de réponse reçu formant des signaux d'ensemble pour le signal de réponse respectif, et
l'obtention des au moins trois éléments d'information vectorielle de prédiction ($V_{12}$, $V_{13}$, $V_{23}$) comprend l'obtention d'une fréquence Doppler de chaque signal de réponse (R) inclus dans la pluralité de paires de signaux de réponse ($R_{12}$, $R_{13}$, $R_{23}$) au moyen d'une autocorrélation entre les signaux d'ensemble des signaux de réponse.

**8.** Appareil à ultrasons pour l'imagerie de vitesse (100) comprenant :

une sonde (110) qui est adaptée pour émettre un signal ultrasonore (T) jusqu'à un point prédéterminé (P(22)) inclus dans un objet et pour recevoir au moins trois signaux de réponse ($R_1$, $R_2$, $R_3$) réfléchis depuis le point prédéterminé (P(22)), chacun des au moins trois signaux de réponse ($R_1$, $R_2$, $R_3$) ayant respectivement une fréquence Doppler et un angle de réception ($\theta_1$, $\theta_2$, $\theta_3$) ;
une unité d'extraction (120) qui est adaptée pour extraire une pluralité de paires de signaux de réponse ($R_{12}$, $R_{13}$, $R_{23}$) en combinant chacun des au moins trois signaux de réponse reçus ($R_1$, $R_2$, $R_3$) avec chacun des autres signaux ($R_1$, $R_2$, $R_3$) ;
une unité d'obtention d'informations vectorielles (130) qui est adaptée pour obtenir au moins trois éléments d'information vectorielle de prédiction ($V_{12}$, $V_{13}$, $V_{23}$), chacun des au moins trois éléments d'information vectorielle de prédiction ($V_{12}$, $V_{13}$, $V_{23}$) indiquant une amplitude de vitesse de prédiction et une direction de vitesse de prédiction du point prédéterminé (P(22)), sur la base d'angles de réception ($\theta_1$, $\theta_2$, $\theta_3$) et de fréquences Doppler des signaux de réponse inclus dans les au moins trois paires de signaux de réponse ($R_{12}$, $R_{13}$, $R_{23}$) ; et
une unité de détermination d'informations vectorielles (140) qui détermine des informations vectorielles bidimensionnelles (V) indiquant une amplitude de vitesse et une direction de vitesse du point prédéterminé, sur la base des au moins trois éléments d'information vectorielle de prédiction ($V_{12}$, $V_{13}$, $V_{23}$).

**9.** Appareil à ultrasons de la revendication 8, dans lequel l'unité de détermination d'informations vectorielles (140) est adaptée pour déterminer les informations vectorielles (V) en utilisant une somme vectorielle linéaire des au moins trois éléments d'information vectorielle de prédiction ($V_{12}$, $V_{13}$, $V_{23}$) .

10. Appareil à ultrasons de la revendication 8, dans lequel l'unité de détermination d'informations vectorielles (140) est adaptée pour déterminer l'une quelconque de la pluralité de directions de vitesse de prédiction incluses dans les au moins trois éléments d'information vectorielle de prédiction ($V_{12}$, $V_{13}$, $V_{23}$) comme la direction de vitesse du point prédéterminé (P(22)), et pour déterminer une somme linéaire de la pluralité d'amplitudes de vitesse de prédiction incluses dans les au moins trois éléments d'information vectorielle de prédiction ($V_{12}$, $V_{13}$, $V_{23}$) comme l'amplitude de vitesse du point prédéterminé (P(22)).

11. Appareil à ultrasons de la revendication 8, dans lequel l'unité de détermination d'informations vectorielles (140) est adaptée pour déterminer des informations vectorielles (V) d'une pluralité de points supplémentaires situés à moins d'une distance prédéfinie du point prédéterminé (P(22)).

12. Appareil à ultrasons de la revendication 11, dans lequel l'unité de détermination d'informations vectorielles (140) est adaptée pour compenser la direction de vitesse du point prédéterminé (P(22)) en appliquant un filtre de lissage à des directions de vitesse de la pluralité de points supplémentaires, et pour déterminer la direction de vitesse compensée comme la direction de vitesse du point prédéterminé (P(22)).

13. Appareil à ultrasons de la revendication 11, dans lequel l'unité de détermination d'informations vectorielles (140) est adaptée pour compenser l'amplitude de vitesse du point prédéterminé (P(22)) en appliquant un filtre de lissage à des amplitudes de vitesse de la pluralité de points supplémentaires, et pour déterminer l'amplitude de vitesse compensée comme l'amplitude de vitesse du point prédéterminé (P(22)).

14. Appareil à ultrasons de la revendication 8, dans lequel la sonde (110) est adaptée pour émettre le signal ultrasonore (T) une pluralité de fois et pour recevoir chacun des au moins trois signaux de réponse ($R_1$, $R_2$, $R_3$) une pluralité de fois, une pluralité du même signal de réponse reçu (R) formant des signaux d'ensemble pour le signal de réponse respectif (R), et l'unité d'obtention d'informations vectorielles (140) est adaptée pour obtenir une fréquence Doppler de chaque signal de réponse (R) inclus dans la pluralité de paires de signaux de réponse ($R_{12}$, $R_{13}$, $R_{23}$) en utilisant une autocorrélation entre les signaux d'ensemble du signal de réponse (R).

15. Support d'enregistrement non transitoire lisible par ordinateur sur lequel est enregistré un programme informatique destiné à exécuter le procédé de l'une quelconque des revendications 1 à 7.

# FIG. 1 (RELATED ART)

# FIG. 2

EP 2 628 449 B1

## FIG. 3

## FIG. 4

19

FIG. 5A

FIG. 5B

FIG. 6

100

140
VECTOR INFORMATION
DETERMINATION UNIT

150

130
VECTOR INFORMATION
OBTAINING UNIT

120
EXTRACTION UNIT

110

FIG. 7A

FIG. 7B

## FIG. 8

```
                    ( START )
                        │
                        ▼
    ┌─────────────────────────────────────────┐
    │      TRANSMIT ULTRASOUND SIGNAL TO       │─── S110
    │  PREDETERMINED POINT INCLUDED IN OBJECT  │
    └─────────────────────────────────────────┘
                        │
                        ▼
    ┌─────────────────────────────────────────┐
    │    RECEIVE AT LEAST THREE RESPONSE       │─── S120
    │  SIGNALS REFLECTED FROM PREDETERMINED    │
    │                 POINT                    │
    └─────────────────────────────────────────┘
                        │
                        ▼
    ┌─────────────────────────────────────────┐
    │  EXTRACT PLURALITY OF RESPONSE SIGNAL    │─── S130
    │                 PAIRS                    │
    └─────────────────────────────────────────┘
                        │
                        ▼
    ┌─────────────────────────────────────────┐
    │  OBTAIN PLURALITY OF PIECES OF           │─── S140
    │        PREDICTION VECTOR INFORMATION     │
    └─────────────────────────────────────────┘
                        │
                        ▼
    ┌─────────────────────────────────────────┐
    │      DETERMINE VECTOR INFORMATION OF     │─── S150
    │           PREDETERMINED POINT            │
    └─────────────────────────────────────────┘
                        │
                        ▼
                    (  END  )
```

## FIG. 9

200

```
              ┌───────────────────────┐  ─ 240
              │      CONTROL UNIT      │
              └───────────────────────┘
                          │        ─ 230
              ┌───────────────────────┐
              │    MEASUREMENT UNIT    │
              └───────────────────────┘
                          │        ─ 220
              ┌───────────────────────┐
              │  REGION DIVIDING UNIT  │
              └───────────────────────┘
                          │
                          │
                         ┌─┐
                       ─ 210
```

## FIG. 10

## FIG. 11

FIG. 12

FIG. 13

# FIG. 14

```
                    ┌─────────┐
                    │  START  │
                    └────┬────┘
                         │
                         ▼
   ┌─────────────────────────────────────────┐
   │ DIVIDE OBJECT INTO PLURALITY OF REGIONS  │── S210
   └─────────────────────┬───────────────────┘
                         │
                         ▼
   ┌─────────────────────────────────────────┐
   │       TRANSMIT ULTRASOUND SIGNAL TO      │── S220
   │           PLURALITY OF REGIONS           │
   └─────────────────────┬───────────────────┘
                         │
                         ▼
   ┌─────────────────────────────────────────┐
   │   RECEIVE RESPONSE SIGNALS REFLECTED     │── S230
   │        FROM PLURALITY OF REGIONS         │
   └─────────────────────┬───────────────────┘
                         │
                         ▼
   ┌─────────────────────────────────────────┐
   │    MEASURE POWER LEVELS OR DOPPLER       │── S240
   │    FREQUENCIES OF RESPONSE SIGNALS       │
   └─────────────────────┬───────────────────┘
                         │
                         ▼
   ┌─────────────────────────────────────────┐
   │        DETERMINE MONITORING REGION       │── S250
   └─────────────────────┬───────────────────┘
                         │
                         ▼
                    ┌─────────┐
                    │   END   │
                    └─────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2003100832 A1
- US 2003212329 A1
- US 4800891 A

**Non-patent literature cited in the description**

- **MANIATIS et al.** *Two-dimensional velocity reconstruction strategies for color flow Doppler ultrasound images,* 1994
- **DUNMIRE et al.** *Cross-beam vector Doppler ultrasound for angle-independent velocity measurements,* 2000
- **BUROV et al.** *Reconstruction of the distribution pattern for the bloodstream velocity vector in the acoustic tomography procedure,* 2006